# FASCICULE DE BREVET EUROPEEN

(11) **EP 1 359 914 B1**
(45) Date de publication et mention de la délivrance du brevet: **30.08.2006**
(21) Numéro de dépôt: 01994896.7
(22) Date de dépôt: 19.12.2001
(51) Int. Cl.: A61K 31/4535, A61P 25/04, A61K 31/445

(54) **NOUVELLE APPLICATION THERAPEUTIQUE D'UN DERIVE DE LA THIENYLCYCLOHEXYLAMINE POUR TRAITER LA DOULEUR**
NEUE THERAPEUTISCHE VERWENDUNG EINES DERIVATES VON THIENYLCYCLOHEXYLAMINE ZUR BEHANDLUNG VON SCHMERZEN
NOVEL THERAPEUTIC USE OF A THIENYLCYCLOHEXYLAMINE DERIVATIVE FOR TREATING PAIN

(30) Priorité: 20.12.2000 FR 0016631
(43) Date de publication de la demande: 12.11.2003
(73) Titulaire: SOCIETE DE CONSEILS DE RECHERCHES ET D'APPLICATIONS SCIENTIFIQUES (S.C.R.A.S.), 75016 Paris (FR)
(72) Inventeur: SIMONNET, Guy, F-33200 Bordeaux (FR); BERNARD d'ARBIGNY, Pierre, F-92400 Courbevoie (FR)
(74) Mandataire: Bourgouin, André
(86) Numéro de dépôt international: PCT/FR2001/004050
(87) Numéro de publication internationale: WO 2002/049647

(56) Documents cités:
- US-A- 5 179 109
- US-A- 5 972 952
- VINCENT, JEAN PIERRE ET AL: "Compared properties of central and peripheral binding sites for phencyclidine" EUR. J. PHARMACOL. (1980), 68(1), 79-82, XP001025576
- TOURNEUR, Y. ET AL: "Phencyclidine blockade of sodium and potassium channels in neuroblastoma cells" BRAIN RES. (1982), 245(1), 154-8, XP001025577

## Description

La présente invention concerne l'utilisation d'une thiénylcyclohexylamine, en association avec d'autres substances à activité pharmaceutique, pour la préparation d'un médicament destiné à prévenir et/ou traiter la douleur et/ou la nociception. L'invention concerne également un produit comprenant une thiénylcyclohexylamine et au moins une substance analgésique, et une composition pharmaceutique le contenant. Ce produit est particulièrement intéressant également pour prévenir les effets facilitateurs de la douleur (hyperalgie) induits paradoxalement par les substances opiacées à la suite de leur effet analgésique.

Par le terme douleur, il faut comprendre "l'expérience désagréable émotionnelle et sensorielle associée à un dommage tissulaire présent ou potentiel ou décrite par le patient en de tels termes" (définition selon l'Internal Association for the study of Pain (IASP)). Par la suite, dans la présente demande, le terme douleur est utilisé indépendamment pour désigner la douleur ou la nociception.

La phencyclidine et certains de ses dérivés tels que le 2-méthyl-1-(1-pipéridinyl)-1-(2-thiényl) cyclohexane ont été testés (European Journal of Pharmacology, 68 (1980) 79-8 ; Brain Research, 245 (1982) 154-158). Cependant l'utilisation du 2-méthyl-1-(1-pipéridinyl)-1-(2-thiényl) cyclohexane avec un analgésique opiacé n'est pas décrite.

L'invention a donc pour objet l'utilisation de la thiénylcyclohexylamine répondant à la formule 2-méthyl-1-(1-pipéridinyl)-1-(2-thiényl) cyclohexane, avec un analgésique agissant sur les récepteurs opiacés pour la préparation d'un médicament destiné à prévenir et/ou traiter la douleur.

La thiénylcyclohexylamine telle que définie ci-dessus, est décrite dans le brevet EP 396734. Compte tenu de l'existence de 2 carbones asymétriques, cette thiénylcyclohexylamine peut être sous forme racémique, de diastéréoisomères ou d'énantiomères substantiellement purs. La préparation des diastéréoisomères de la 1-thiénylcyclohexylamine est décrite dans le brevet US 5972952.

L'analgésique agissant sur les récepteurs opiacés sera utilisé à forte dose à l'occasion d'acte chirugical ou de manière répétée lors de la prise en charge de douleurs rebelles ou chroniques.

Parmi les analgésiques opiacés couramment utilisés, on peut citer le fentanyl, sufentanil, alfentanil, codéine, péthidine, rémifentanil, morphine, tramadol, buprénorphine, nalbuphine, sulfate de morphine, chlorhydrate d'hydromorphone, sulfate de morphine enrobé.

L'invention a également pour objet un produit comprenant la thiénylcyclohexylamine répondant à la formule 2-méthyl-1-(1-pipéridinyl)-1-(2-thiényl) cyclohexane sous forme racémique, de diastéréoisomères ou d'énantiomères substantiellement purs, et au moins une substance analgésique en tant que produit de combinaison pour une utilisation simultanée, séparée ou étalée dans le temps pour traiter et/ou prévenir la douleur. L'analgésique est un analgésique agissant sur les récepteurs opiacés, et très préférentiellement, l'analgésique agissant sur les récepteurs opiacés est un analségique opiacé.

Préférentiellement, l'analgésique opiacé associé à la thiénylcyclohexylamine est choisi parmi fentanyl, alfentanil, codéine, péthidine, rémifentanyl, morphine, tramadol, buprénorphine, nalbuphine, sulfate de morphine, chlorhydrate d'hydromorphone, sulfate de morphine enrobé, et très préférentiellement l'analgésique opiacé est le fentanyl.

L'invention a plus particulièrement pour objet, à titre de médicament, un produit contenant la thiénylcyclohexylamine tel que défini ci-dessus, sous forme racémique, de diastéréoisomères ou d'énantiomères substantiellement purs, associée avec au moins une substance analgésique. L'invention a plus particulièrement pour objet également une composition pharmaceutique contenant, à titre de principe actif, un médicament tel que défini ci-dessus.

La thiénylcyclohexylamine telle que définie ci-dessus, peut être administrée à une dose comprise entre 0,001 et 10 mg/kg, de préférence entre 0,01 et 1 mg/kg. Les substances qui lui sont éventuellement associées, substances analgésiques opiacées, connues en pharmacologie, sont administrées aux doses habituellement conseillées dans les domaines de la douleur et de la nociception.

La thiénylcyclohexylamine telle que définie ci-dessus ainsi que les substances à activité pharmaceutique qui lui sont associées, peuvent être administrées par les voies classiques d'administration telle que orale, intramusculaire, intrapéritonéale, sous-cutanée ou intraveineuse. Elles peuvent être administrées simultanément ou séparément, par des voies d'administration identiques ou différentes. De préférence, la thiénylcyclohexylamine est administrée par voie intraveineuse ou sous-cutanée et les substances à activité pharmaceutique qui lui sont associées sont administrées par voie intraveineuse ou sous-cutanée. La gacyclidine peut être administrée avant l'administration de la substance analgésique agissant sur les récepteurs opiacés.

Enfin l'invention a également pour objet l'utilisation de la thiénylcyclohexylamine telle que définie ci-dessus, pour la préparation d'un médicament susceptible de prévenir les hyperalgies et/ou allodynies induites par un analgésique agissant sur les récepteurs opiacés. En effet, comme cela est présenté dans la partie expérimentale (cf. phase 2), le traitement par le fentanyl, analgésique opiacé très largement utilisé en milieu hospitalier à l'occasion d'actes chirurgicaux, induit une allodynie pendant plusieurs jours. Cette allodynie est totalement prévenue par la thiénylcyclohexylamine selon l'invention : une seule injection (30 minutes avant l'analgésique) même à la dose de 0,1 mg/kg qui ne provoque pas *per se* d'effet analgésique à cette dose, prévient totalement cette allodynie de plusieurs jours. Par ailleurs, la thiénylcyclohexylamine testée ne présente pas d'effet psychomoteur aux doses efficaces de 0,1 et 0,3 mg/kg. De préférence, la thiénylcyclohexylamine est administrée avant la substance opiacée. De préférence également, la thiénylcyclohexylamine est administrée à une dose inférieure à 5 mg/kg, et très préférentiellement à une dose inférieure à 0,2 mg/kg.

L'administration de la 1-[cis-2-méthyl-1-(2-thiényl)cyclohexyl]pipéridine (gacyclidine), notamment en association, facilite le sevrage ce qui a pour conséquence une limitation de la durée d'hospitalisation et un retour plus rapide à une vie normale du point de vue professionnel et social.

### PARTIE EXPÉRIMENTALE :

### Etude pharmacologique

Il s'agit d'étudier chez le rat, les effets de la 1-[cis-2-méthyl-1-(2-thiényl)cyclohexyl]pipéridine (gacyclidine), sur les effets analgésiques immédiats du fentanyl mais aussi sur les effets facilitateurs de la douleur (hyperalgie) induits par l'administration de cet analgésique opiacé réalisé en plusieurs bolus par voie veineuse de manière à se rapprocher de son utilisation en clinique chirurgicale humaine.

Toutes les expériences sont réalisées sur des rats Sprague-Dawley d'un poids de 350-400 g ; chaque phase expérimentale est réalisée selon un schéma similaire : chaque phase expérimentale est réalisée sur 6 groupes de 12 rats dont un lot d'animaux témoins. Le test de mesure du seuil nociceptif retenu est le Test de Randall-Seltto modifié selon Kayser et al. (1990) (Kayser V., Basbaum A.I. et Guilbaud G., Deafferentation in the rat increase mechanical nociceptive threshold in the innervated limbs ; Brain research (1999), 508, 329-332), utilisant un stimulus mécanique d'intensité croissante (exprimée en grammes), la réponse évoquée retenue étant le cri de l'animal.

Le schéma général d'une phase expérimentale est le suivant :
- arrivage et stabulation des animaux : 4 j ;
- préparation des animaux leur permettant de se familiariser avec l'expérimentateur et les conditions et mesures expérimentales afin d'éviter tout biais de mesure pouvant être induit par le stress: 14 j ;
- préparation et mise en place des cathéters : 9 j ;
- repos post-opératoire (avec traitement antibiotique) et détermination de la stabilité des mesures expérimentales (seuil nociceptif de base) : 4j ;
- tests de réponse aux différents principes actifs : 9 j.

Les 3 doses retenues de gacyclidine : 0,1, 0,3 et 1 mg/kg, sont injectées par voie veineuse à l'aide du cathéter posé dans une veine jugulaire.

L'ensemble des résultats est analysé selon un test ANOVA.

### Phase 1 : étude des effets propres de la gacyclidine sur le seuil nociceptif chez le rat. (Etude comparative)

### Protocole - Phase 1

3 séries expérimentales comprenant chacune 2 lots d'animaux de 12 rats sont constituées.
1° série expérimentale:
   1° lot : les animaux reçoivent une injection de sérum physiologique.
   2° lot : les animaux reçoivent une dose de 0,1 mg/kg de gacyclidine.
2° série expérimentale :
   1° lot : les animaux reçoivent une injection de sérum physiologique.
   2° lot : les animaux reçoivent une dose de 0,3 mg/kg de gacyclidine.
3° série expérimentale :
   1 ° lot : les animaux reçoivent une injection de sérum physiologique.
   2° lot : les animaux reçoivent une dose de 1 mg/kg de gacyclidine.

L'ensemble des animaux des 3 séries expérimentales est préparé au cours d'une même phase. Le jour d'administration de la gacyclidine (ou du sérum physiologique) pour chaque série est décalé seulement de 3 jours afin de suivre l'évolution du seuil nociceptif pendant plusieurs jours suivant l'administration des substances pharmaceutiques. La mesure du seuil nociceptif est réalisé pendant au moins 4 heures après injection de gacyclidine, à raison d'une mesure toutes les 30 minutes puis de manière quotidienne pendant au moins une semaine.

### Résultats - Phase 1

La gacyclidine induit un effet analgésique pendant les 30 premières minutes à la dose de 0,3 mg/kg, et pendant la première heure à la dose de 1 mg/kg. De manière intéressante, la gacyclidine n'induit d'effets moteurs qu'à la plus forte dose utilisée (1 mg/kg).

### Phase 2 : Etude des capacités de la gacyclidine à s'opposer à l'hyperalgie induite par la naloxone lorsque celle-ci est administrée pendant l'effet analgésique du fentanyl.

### Protocole - Phase 2

Le fentanyl est administré selon un protocole "mimant" son usage en chirurgie : 4 injections intraveineuses consécutives (toutes les 15 minutes) d'une dose de 40 µg/kg.

Chaque animal reçoit 3 types d'injection :
la première injection (saline ou gacyclidine) est faite 30 minutes après la mesure du seuil nociceptif de base,
la série d'injections de sérum physiologique ou de fentanyl (4 injections intraveineuses consécutives toutes les 15 minutes) est commencée 30 minutes après la première injection de sérum physiologique ou de gacyclidine,
la troisième injection (naloxone dont le rôle est de bloquer les récepteurs opiacés) est réalisée 10 minutes après la dernière injection de fentanyl ; l'effet de la naloxone sur le seuil nociceptif est apprécié 5 minutes après son injection, puis à 20 et 35 minutes, puis toutes les 30 minutes pendant deux heures.

3 séries expérimentales comprenant chacune 2 lots d'animaux de 12 rats sont constituées.
1° série expérimentale :
   1° lot : les animaux reçoivent successivement sérum physiologique, fentanyl (4 x 40 µg/kg) puis naloxone (1 mg/kg s.c.).
   2° lot : les animaux reçoivent successivement gacyclidine (0,1 mg/kg), fentanyl (4 x 40 µg/kg) puis naloxone (1 mg/kg s.c.).
2° série expérimentale :
   1° lot : les animaux reçoivent successivement sérum physiologique, fentanyl (4 x 40 µg/kg) puis naloxone (1 mg/kg s.c.).
   2° lot : les animaux reçoivent successivement gacyclidine (0,3 mg/kg), fentanyl (4 x 40 µg/kg) puis naloxone (1 mg/kg s.c.).
3° série expérimentale :
   1° lot : les animaux reçoivent successivement sérum physiologique, fentanyl (4 x 40 µg/kg) puis naloxone (1 mg/kg s.c.).
   2° lot : les animaux reçoivent successivement gacyclidine (1 mg/kg), fentanyl (4 x 40 µg/kg) puis naloxone (1 mg/kg s.c.).

L'ensemble des animaux des 3 séries expérimentales est préparé au cours d'une même phase. Le jour d'administration des substances pharmacologiques pour chaque série est décalé seulement de 3 jours afin de pouvoir suivre l'évolution du seuil nociceptif de manière quotidienne pendant plusieurs jours (au moins une semaine) suivant l'administration de ces substances, afin de pouvoir évaluer l'amplitude et la durée de l'hyperalgie induite par le fentanyl. La mesure du seuil nociceptif est réalisée pendant au moins 4 heures après injection de fentanyl, à raison d'une mesure toutes les 30 minutes, l'effet particulier de la naloxone étant mesuré 5 minutes après l'administration de cet antagoniste des récepteurs opiacés réalisée après la dernière injection de fentanyl.

### Résultats - Phase 2

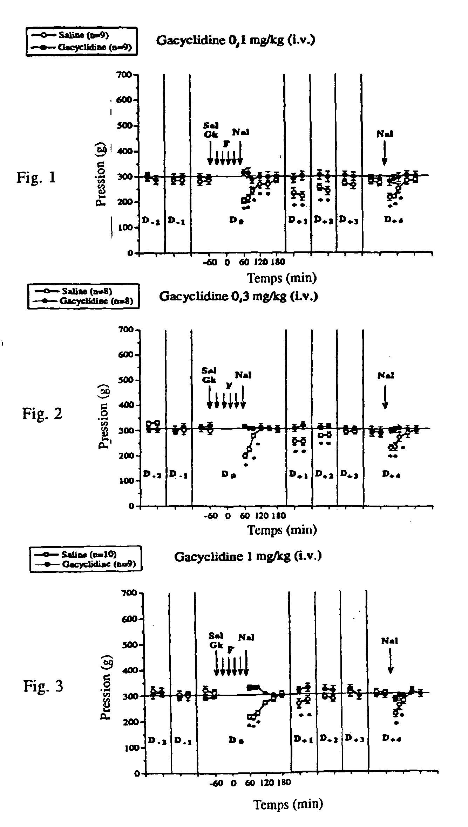

Les résultats sont présentés dans les graphiques ci-dessous (fig. 1-3).

La naloxone, injectée durant l'analgésie induite par le fentanyl, provoque une baisse importante du seuil nociceptif, au dessous des valeurs de base, confirmant que le fentanyl active un système facilitateur de la nociception. Cette activation est prévenue chez les animaux prétraités par la gacyclidine. Il est important de relever que même la dose la plus faible de gacyclidine (0,1 mg/kg) qui n'induit *per se* ni effet analgésique ni effet moteur, prévient déjà totalement cet effet.

### Phase 3 : Evaluation de l'effet potentialisateur de la gacyclidine sur l'effet analgésique de fentanyl.

Le fentanyl est administré selon un protocole "mimant" son usage en chirurgie : 4 injections intraveineuses consécutives (toutes les 15 minutes) d'une dose de 40 µg/kg.

Chaque animal reçoit 2 types d'injection :
la première injection (saline ou gacyclidine) est faite 30 minutes après la mesure du seuil nociceptif de base,
la série d'injections de sérum physiologique ou de fentanyl (4 injections consécutives de 40 µg/kg de fentanyl toutes les 15 minutes) est commencée 30 minutes après cette injection de sérum physiologique ou de gacyclidine.

### Protocole - Phase 3

3 séries expérimentales comprenant chacune 2 lots d'animaux de 12 rats sont constituées.
1° série expérimentale :
   1° lot : les animaux reçoivent successivement sérum physiologique puis fentanyl (4 x 40 µg/kg).
   2° lot: les animaux reçoivent successivement gacyclidine (0,1 mg/kg) puis fentanyl (4 x 40 µg/kg).
2° série expérimentale :
   1° lot : les animaux reçoivent successivement sérum physiologique puis fentanyl (4 x 40 µg/kg)
   2° lot : les animaux reçoivent successivement gacyclidine (0,3 mg/kg) puis fentanyl (4 x 40 µg/kg).
3° série expérimentale :
   1° lot : les animaux reçoivent successivement sérum physiologique puis fentanyl (4 x 40 µg/kg).
   2° lot : les animaux reçoivent successivement gacyclidine (1 mg/kg) puis fentanyl (4 x 40 µg/kg).

L'ensemble des animaux des 3 séries expérimentales est préparé au cours d'une même phase. Le jour de l'administration de la gacyclidine (ou de sérum physiologique) pour chaque série est décalé seulement de 3 jours afin de suivre l'évolution du seuil nociceptif (en particulier détecter toute baisse correspondant à l'hyperalgie induite par le fentanyl) pendant plusieurs jours suivant l'administration des substances pharmacologiques. La mesure expérimentale du seuil nociceptif est réalisée pendant au moins 4 heures après la dernière injection de fentanyl à raison d'une mesure toutes les 30 minutes puis de manière quotidienne pendant au moins une semaine.

### Résultats - Phase 3

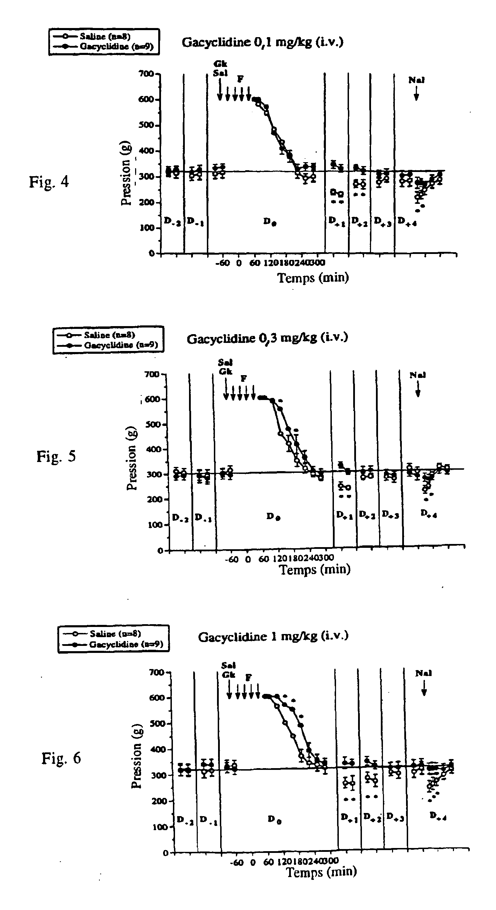

Les résultats sont présentés dans les graphiques ci-dessous (fig. 4-6).

Aux doses de 0,3 et 1 mg/kg, la gacyclidine potentialise l'effet analgésique du fentanyl. A toutes les doses (0,1, 0,3 et 1 mg/kg), la gacyclidine prévient l'allodynie de longue durée confirmant les résultats de la phase 2.

Dans les graphiques ci-dessous correspondant aux phases 2 et 3, la valeur de la pression (exprimée en gramme) sur la patte de l'animal, la réponse retenue étant le cri de l'animal, est mesurée en fonction du temps (exprimé en minutes). Pour chaque phase, il y a 3 graphiques correspondant respectivement aux doses de 0,1, 0,3 et 1 mg/kg de gacyclidine.

Les abréviations utilisées sont les suivantes : Sal : saline ; GK : gacyclidine ; F : fentanyl ; Nal : naloxone ; * : p< 0,05.

## Revendications

1. Utilisation d'un dérivé de la thiénylcyclohexylamine répondant à la formule 2-méthyl-1-(1-pipéridinyl)-1-(2-thiényl) cyclohexane, sous forme racémique, de diastéréoisomères ou d'énantiomères substantiellement purs, pour la préparation d'un médicament destiné à traiter et/ou prévenir la douleur, **caractérisée en ce que** le dérivé de la thiénylcyclohexylamine est associée à un analgésique agissant sur les récepteurs opiacés.

2. Utilisation selon la revendication 1, **caractérisée en ce que** la substance analgésique agissant sur les récepteurs opiacé est un analgésique opiacé choisi parmi fentanyl, alfentanil, codéine, péthidine, rémifentanyl, morphine, tramadol, buprénorphine, nalbuphine, sulfate de morphine, chlorhydrate d'hydromorphone, sulfate de morphine enrobé.

3. Utilisation selon l'une des revendications précédentes, **caractérisée en ce que** la substance analgésique agissant sur les récepteurs opiacé est le fentanyl.

4. Produit comprenant le dérivé de la thiénylcyclohexylamine répondant à la formule 2-méthyl-1-(1-pipéridinyl)-1-(2-thiényl) cyclohexane sous forme racémique, de diastéréoisomères ou d'énantiomères substantiellement purs, et au moins une substance analgésique agissant sur les récepteurs opiacés en tant que produit de combinaison pour une utilisation simultanée, séparée ou étalée dans le temps pour traiter et/ou prévenir la douleur.

5. Produit selon la revendication 4, **caractérisé en ce que** la substance analgésique agissant sur les récepteurs opiacés est un analgésique opiacé choisi parmi fentanyl, alfentanil, codéine, péthidine, rémifentanyl, morphine, tramadol, buprénorphine, nalbuphine, sulfate de morphine, chlorhydrate d'hydromorphone, sulfate de morphine enrobé.

6. Produit selon l'une des revendications 4 à 5, **caractérisé en ce que** la substance analgésique agissant sur les récepteurs opiacés est le fentanyl.

7. Produit selon l'une des revendications 4 à 6, **caractérisé en ce que** le dérivé de la thiénylcyclohexylamine correspond à la 1-[cis-2-méthyl-1-(2-thiényl)cyclohexyl] pipéridine.

8. A titre de médicament, un produit tel que défini à l'une des revendications 4 à 7.

9. Compositions pharmaceutiques contenant, à titre de principe actif, un médicament tel que défini à la revendication 8.

10. Utilisation d'un dérivé de la thiénylcyclohexylamine répondant à la formule 2-méthyl-1-(1-pipéridinyl)-1-(2-thiényl) cyclohexane, sous forme racémique, de diastéréoisomères ou d'énantiomères substantiellement purs, pour la préparation d'un médicament destiné à prévenir les hyperalgies et/ou allodynies induites par un analgésique agissant sur les récepteurs opiacés.

11. Utilisation selon la revendication 10 **caractérisée en ce que** la thiénylcyclohexylamine est administrée avant la substance opiacée.

12. Utilisation selon l'une des revendications 10 ou 11, **caractérisée en ce que** le dérivé de la thiénylcyclohexylamine est administrée à une dose inférieure à 5 mg/kg, et préférentiellement inférieure à 2 mg/kg.

13. Utilisation selon l'une des revendications 1 à 3 ou 10 à 12, **caractérisée en ce que** le dérivé de la thiénylcyclohexylamine correspond à la 1-[cis-2-méthyl-1-(2-thiényl)cyclohexyl] pipéridine.

## Claims

1. Use of a thienylcyclohexylamine derivative corresponding to the formula 2-methyl-1-(1-piperidinyl)-1-(2-thienyl) cyclohexane, in racemic form, or in the form of essentially pure diastereoisomers or enantiomers, for the preparation of a medicament intended to treat and/or prevent pain, **characterized in that** the thienylcyclohexylamine is combined with an analgesic acting on the opiate receptors.

2. Use according to claim 1, **characterized in that** the analgesic substance acting on the opiate receptors is an opiate analgesic chosen from fentanyl, alfentanil, codeine, pethidine, remifentanyl, morphine, tramadol, buprenorphine, nalbuphine, morphine sulphate, hydromorphone hydrochloride, coated morphine sulphate,

3. Use according to any of the preceding claims, **characterized in that** the analgesic substance acting on the opiate receptors is fentanyl.

4. Product comprising a thienylcyclohexylamine derivative corresponding to the formula 2-methyl-1-(1-piperidinyl)-1-(2-thienyl) cyclohexane in racemic form, or in the form of essentially pure diastereoisomers or enantiomers, and at least one analgesic substance acting on the opiate receptors as a combination product for simultaneous or separate use, or use spread out over time in order to treat and/or prevent pain.

5. Product according to claim 4, **characterized in that** the analgesic substance acting on the opiate receptors is an opiate analgesic chosen from fentanyl, alfentanil, codeine, pethidine, remifentanyl, morphine, tramadol, buprenorphine, nalbuphine, morphine sulphate, hydromorphone hydrochloride, coated morphine sulphate

6. Product according to one of claim 4 or 5, **characterized in that** the analgesic substance acting on the opiate receptors is fentanyl.

7. Product according to one of claims 4 to 6, **characterized in that** the thienylcyclohexylamine derivative corresponds to 1-[cis-2-methyl-1-(2-thienyl)cyclohexyl] piperidine.

8. As a medicament, a product as defined in one of claims 4 to 7.

9. Pharmaceutical compositions containing, as active ingredient, a medicament as defined in claim 8.

10. Use of a thienylcyclohexylamine derivative corresponding to the formula 2-methyl-1-(1-piperidinyl)-1-(2-thienyl) cyclohexane, in racemic form, or in the form of essentially pure diastereoisomers or enantiomers, for the preparation of a medicament intended to prevent hyperalgias and/or allodynias induced by an analgesic acting on the opiate receptors.

11. Use according to claim 10, **characterized in that** the thienylcyclohexylamine is administered before the opiate substance.

12. Use according to one of claims 10 or 11, **characterized in that** the thienylcyclohexylamine derivative is administered at a dose of less than 5 mg/kg, and preferably less than 2 mg/kg.

13. Use according to one of claims 1 to 3 or 10 to 12, **characterized in that** the thienylcyclohexylamine derivative corresponds to 1-[cis-2-methyl-1-(2-thienyl)cyclohexyl] piperidine.

## Patentansprüche

1. Verwendung eines Thienylcyclohexylamin-Derivats der Formel 2-Methyl-1-(1-piperidinyl)-1-(2-thienyl)-cyclohexan in racemischer Form, in Form von im Wesentlichen reinen Diastereoisomeren oder Enantiomeren für die Herstellung eines für die Behandlung und/oder Prävention von Schmerzen bestimmten Medikaments, **dadurch gekennzeichnet, dass** das Thienylcyclohexylamin-Derivat mit einem auf die Opiat- bzw. Opioidrezeptoren einwirkenden Analgetikum kombiniert ist.

2. Verwendung nach Anspruch 1, **dadurch gekennzeichnet, dass** die auf die Opiat- bzw. Opioidrezeptoren einwirkende analgetische Substanz ein Opiat- bzw. Opioidanalgetikum ist, das aus Fentanyl, Alfentanil, Codein, Pethidin, Remifentanyl, Morphin, Tramadol, Buprenorphin, Nalbuphin, Morphinsulfat, Hydromorphonchlorhydrat, umhülltes Morphinsulfat ausgewählt ist.

3. Verwendung nach einem der vorhergehenden Ansprüche, **dadurch gekennzeichnet, dass** die auf die Opiat- bzw. Opioidrezeptoren einwirkende analgetische Substanz Fentanyl ist.

4. Produkt, das das Thienylcyclohexylamin-Derivat der Formel 2-Methyl-1-(1-piperidinyl)-1-(2-thienyl)-cyclohexan in racemischer Form, in Form von im Wesentlichen reinen Diastereoisomeren oder Enantiomeren und mindestens eine auf die Opiat- bzw. Opioidrezeptoren einwirkende analgetische Substanz umfasst, als Kombinationsprodukt für eine gleichzeitige, getrennte oder zeitlich verteilte Verwendung für die Behandlung und/oder Prävention von Schmerzen.

5. Produkt nach Anspruch 4, **dadurch gekennzeichnet, dass** die auf die Opiat- bzw. Opioidrezeptoren einwirkende analgetische Substanz ein Opiat- bzw. Opioidanalgetikum ist, das aus Fentanyl, Alfentanil, Codein, Pethidin, Remifentanyl, Morphin, Tramadol, Buprenorphin, Nalbuphin, Morphinsulfat, Hydromorphonchlorhydrat, umhülltes Morphinsulfat ausgewählt ist.

6. Produkt nach einem der Ansprüche 4 bis 5, **dadurch gekennzeichnet, dass** die auf die Opiat- bzw. Opioidrezeptoren einwirkende analgetische Substanz Fentanyl ist.

7. Produkt nach einem der Ansprüche 4 bis 6, **dadurch gekennzeichnet, dass** das Thienylcyclohexylamin-Derivat dem 1-[cis-2-Methyl-1-(2-thienyl)cyclohexyl]piperidin entspricht.

8. Ein Produkt, wie es in einem der Ansprüche 4 bis 7 definiert ist, in Form eines Medikaments.

9. Pharmazeutische Zusammensetzungen, die als Wirkstoff ein Medikament enthalten, wie es in Anspruch 8 definiert ist.

10. Verwendung eines Thienylcyclohexylamin-Derivats der Formel 2-Methyl-1-(1-piperidinyl)-1-(2-thienyl)-cyclohexan in racemischer Form, in Form von im Wesentlichen reinen Diastereoisomeren oder Enantiomeren für die Herstellung eines Medikaments, das für die Prävention von Hyperalgien und/oder Allodynien bestimmt ist, die durch ein auf die Opiat- bzw. Opioidrezeptoren einwirkendes Analgetikum induziert werden.

11. Verwendung nach Anspruch 10, **dadurch gekennzeichnet, dass** das Thienylcyclohexylamin vor der Opiat- bzw. Opioidsubstanz verabreicht wird.

12. Verwendung nach einem der Ansprüche 10 oder 11, **dadurch gekennzeichnet, dass** das Thienylcyclohexylamin-Derivat in einer Dosis von weniger als 5 mg/kg und vorzugsweise von weniger als 2 mg/kg verabreicht wird.

13. Verwendung nach einem der Ansprüche 1 bis 3 oder 10 bis 12, **dadurch gekennzeichnet, dass** das Thienylcyclohexylamin-Derivat dem 1-[cis-2-Methyl-1-(2-thienyl)cyclohexyl]-piperidin entspricht.
